# FASCICULE DE BREVET EUROPEEN

(11) **EP 2 059 161 B1**
(45) Date de publication et mention de la délivrance du brevet: **19.12.2012**
(21) Numéro de dépôt: 07823454.9
(22) Date de dépôt: 29.08.2007
(51) Int. Cl.: A47L 15/42, A61L 2/18, A61L 2/24, E06B 5/00, G21K 5/10, A61L 2/28

(54) **MACHINE A LAVER DES INSTRUMENTS MEDICAUX ET/OU CHIRURGICAUX**
WASCHMASCHINE FÜR MEDIZINISCHE UND/ODER CHIRURGISCHE INSTRUMENTE
MACHINE FOR WASHING SURGICAL AND/OR MEDICAL INSTRUMENTS

(30) Priorité: 08.09.2006 FR 0607880
(43) Date de publication de la demande: 20.05.2009
(73) Titulaire: Getinge Lancer, 31170 Tournefeuille (FR)
(72) Inventeur: BRIEUSSEL, Jean-Marie, F-31500 Toulouse (FR); FOGLIANI, NATHALIE, épouse GUILLAUD, F-31270 Frouzins (FR)
(74) Mandataire: Cabinet BARRE LAFORGUE & associés
(86) Numéro de dépôt international: PCT/FR2007/001409
(87) Numéro de publication internationale: WO 2008/029012

(56) Documents cités:
- EP-A- 1 340 512
- WO-A-2006/015934
- BE-A- 644 545
- DE-A1- 3 240 284
- FR-A- 2 843 028
- US-A- 4 042 805

## Description

L'invention concerne une machine à laver des instruments médicaux et/ou chirurgicaux, tels que des fibroscopes, des endoscopes, des sondes d'échocardiographie, des sondes d'échographie, et tout autre instrument requérant un lavage avant chaque utilisation.

Ces instruments médicaux et/ou chirurgicaux requièrent un lavage minutieux et systématique avant chaque utilisation de manière à prévenir tout risque de contamination d'un patient sur lequel ces instruments vont être utilisés. Typiquement, un tel lavage nettoie, rince, désinfecte et sèche l'intérieur et l'extérieur des appareils de manière à éliminer les matières organiques (sang, selles, sécrétions respiratoires, etc.) et les germes éventuellement présents sur, ou dans, ces appareils.

Il existe de nombreuses machines à laver désinfectanteS destinées au lavage d'instruments à usage médical et/ou chirurgical.

Les machines de lavage/désinfection font l'objet de normes sévères concernant notamment leur type et leur utilisation. En particulier, les normes EN ISO 15883-1, 15883-2, 15883-3, 15883-4, et 15883-5 imposent la mise en oeuvre de moyens de traçabilité permettant d'indiquer clairement si le cycle s'est entièrement déroulé ou non, afm d'autoriser ou non l'utilisation des instruments traités. Les machines doivent en outre être équipées d'un système de verrouillage de porte qui interdit l'accès à la charge (instrument) pendant le cycle de traitement. Il est en général possible d'arrêter le cycle de traitement avant la fin de ce dernier, et d'accéder ainsi à la charge. Mais, dans ce cas, le cycle des traitements n'est pas validé et les instruments doivent subir un nouveau cycle de traitement.

Dans tout le texte, sauf indication contraire, les termes « lavage », « laver » et leurs dérivés font référence à tout traitement comprenant au moins une étape effectuée sur tout ou partie d'un instrument, à l'extérieur et/ou à l'intérieur d'un instrument, et choisie dans le groupe formé des étapes de : pré-nettoyage (rinçage initial) ; nettoyage (mécanique et/ou par solution chimique) ; rinçage ; désinfection ; stérilisation ; soufflage ; séchage.

Il existe deux configurations connues de machines à laver les instruments médicaux et/ou chirurgicaux :
- les machines dotées d'une seule porte de chargement/déchargement des instruments (cf. par exemple FR 2806286) ;
- les machines dotées de deux portes distinctes pour chaque cuve de lavage, l'une pour le chargement des instruments à laver, l'autre pour le déchargement des instruments lavés ; ces machines sont placées à la traversée d'une cloison avec la porte de chargement ouvrant d'un côté de la cloison sur une zone « sale » (par exemple une salle de prélavage et de chargement d'une pluralité de machines à laver), alors que la porte de déchargement ouvre de l'autre côté de la cloison sur une zone « propre » (par exemple une salle de conditionnement des instruments lavés et/ou de chargement d'autoclaves de stérilisation pour les instruments thermorésistants).

Les machines de lavage des instruments médicaux et/ou chirurgicaux sont en général également dotées d'automatismes de commande qui gèrent l'ouverture et la fermeture de la porte ou des portes. Par exemple, dans le cas des machines dotées de deux portes, en cas d'arrêt du cycle de traitement avant la fin, l'accès à la charge de la machine ne peut se faire que du côté de la porte de chargement.

Les machines dotées de deux portes distinctes par cuve présentent une plus grande sûreté de fonctionnement dans la mesure où les instruments lavés ne circulent jamais dans un espace dans lequel des instruments non lavés peuvent également circuler. Elles permettent ainsi en particulier la mise en oeuvre du principe dit « de la marche en avant » permettant d'éviter que des instruments lavés puissent venir en contact avec des instruments non lavés à un moment quelconque du cycle de stockage, d'utilisation ou de traitement des instruments. Néanmoins, ces machines conçues pour être agencées à la traversée d'une paroi présentent l'inconvénient d'un coût élevé, d'un encombrement important et de nécessiter une infrastructure et une logistique spécifiques à leur mise en oeuvre (deux salles différentes, dispositions de la machine à la traversée d'une cloison, doublement du personnel...).

Le document DE 3240284 A1 décrit une machine à laver selon le préambule de la revendication 1.

Dans ce contexte, l'invention vise à proposer une machine à laver des instruments médicaux et/ou chirurgicaux qui présentent les avantages combinés des machines connues dotées d'une porte unique et des machines connues à deux portes sans présenter néanmoins les inconvénients ni des unes ni des autres.

L'invention vise en particulier à proposer une telle machine qui permette la mise en oeuvre du principe de la marche en avant, tout en présentant un coût similaire à celui d'une machine connue à porte unique, et inférieur à celui des machines à deux portes distinctes par cuve, et ce aussi bien en ce qui concerne le coût de fabrication de la machine elle-même que celui lié à son installation et à son utilisation.

L'invention vise de surcroît à proposer une telle machine qui soit susceptible, sans intervention physique sur la machine elle-même, par simple modification d'un paramètre enregistré, de faire fonctionner cette machine soit avec un accès sur deux côtés distincts de la machine, soit avec un accès d'un seul côté de la machine, de façon à permettre ainsi une grande souplesse d'utilisation et un fonctionnement évolutif de la machine.

L'invention vise également à proposer une telle machine qui présente une sûreté de fonctionnement renforcée, notamment vis-à-vis des risques de contamination lors des opérations de chargement/déchargement.

Pour ce faire, l'invention concerne une machine à laver des instruments médicaux et/ou chirurgicaux selon la revendication 1.

Une machine à laver selon l'invention comprend, pour chaque cuve, une ouverture d'accès unique et une porte unique. Dans un mode de réalisation avantageux, la machine est dotée d'une seule cuve, et donc d'une seule porte de chargement et de déchargement. Rien n'empêche cependant de réaliser une même machine rassemblant plusieurs cuves de lavage, chaque cuve étant dotée d'une porte unique de chargement/déchargement. Dans cette variante, toutes les ouvertures des portes sont orientées sur un même côté de chargement de la machine, et toutes les ouvertures de déchargement sont orientées sur un même côté opposé de déchargement de la machine.

Pour chaque cuve de lavage, la porte montée sur une articulation bilatérale selon l'invention permet de définir un côté de chargement de la cuve - notamment de la machine - et un côté de déchargement de cuve - notamment de la machine-. De plus, une articulation bilatérale permet une ouverture bilatérale de la porte qui permet un accès à la cuve du côté ouvert et interdit l'accès à la cuve du côté opposé. Dès lors, une machine selon l'invention peut être disposée par exemple entre une salle d'opération et une salle de préparation, le côté de chargement étant accessible depuis la salle de préparation et le côté de déchargement étant accessible depuis la salle d'opération. De cette manière, tout instrument accessible au chirurgien en salle d'opération est nécessairement un instrument propre qui a subit un cycle de lavage. Si le cycle de lavage n'est pas effectué, la porte ne peut être ouverte du côté de déchargement. Il est à noter de surcroît que cette sécurité d'accès est obtenue avec une seule porte, et donc avec une machine moins coûteuse qu'une machine connue dotée de deux portes distinctes par cuve, et sans même nécessiter une infrastructure lourde. Par exemple, une machine selon l'invention peut être utilisée avec une grande sûreté de fonctionnement, selon le principe dit de la marche en avant, mais sans nécessiter d'être interposée à la traversée d'une cloison. En effet, l'articulation bilatérale de la porte permet d'éviter toute erreur lorsque cette dernière est ouverte, et la porte elle-même constitue, en position ouverte, une séparation entre les zones propres et sales. Rien n'empêche cependant de placer une machine selon l'invention au niveau d'une traversée de cloison.

Avantageusement, une machine selon l'invention est aussi caractérisée en ce qu'elle comprend :
- des moyens de verrouillage à au moins deux états de ladite porte, un état verrouillé dans lequel lesdits moyens de verrouillage sont adaptés pour verrouiller ladite porte en position de lavage de telle sorte qu'il soit impossible d'ouvrir ladite porte au cours d'un cycle de lavage, et un état déverrouillé dans lequel lesdits moyens de verrouillage sont adaptés pour autoriser une ouverture de ladite porte,
- une unité de commande desdits moyens de verrouillage adaptée pour commander le verrouillage et le déverrouillage des moyens de verrouillage.

Une machine à laver selon l'invention équipée de moyens de verrouillage et d'une unité de commande de ces moyens de verrouillage permet d'assurer une gestion rigoureuse des cycles de lavage. En particulier, une machine à laver selon l'invention peut garantir que l'accès à la cuve de la machine n'est possible que lorsqu'un cycle de lavage a été effectué. De plus, une machine à laver selon l'invention, équipée d'une unité de commande, peut imposer le verrouillage des moyens de verrouillage de manière à interdire l'accès à la cuve dès que la porte est fermée et autoriser le déverrouillage des moyens de verrouillage notamment si un cycle de lavage sans échec a eu lieu.

Avantageusement, une unité de commande selon l'invention est également adaptée pour interrompre, sur commande d'un opérateur, un cycle de lavage en cours. Dans ce cas, l'unité de commande autorise uniquement une ouverture de la porte du côté de chargement de la machine.

Une unité de commande selon l'invention peut être de tout type connu. Une telle unité de commande peut comprendre des moyens de programmation de cette unité de commande. Une telle unité de commande peut comprendre des moyens analogiques ou des moyens numériques ou une combinaison de moyens analogiques et de moyens numériques.

Avantageusement, une machine à laver selon l'invention comprend, associés à l'unité de commande :
- au moins un capteur de position de ladite porte, dit capteur de porte, adapté pour détecter une position ouverte de ladite porte et une position fermée de ladite porte,
- au moins un capteur de présence d'instruments médicaux et/ou chirurgicaux dans ladite cuve, dit capteur de cuve,
- au moins un témoin de lavage à deux états, un état lavé correspondant à une fin de cycle de lavage, et un état à laver, correspondant à un cycle de lavage à effectuer ou en cours.

Une machine à laver équipée de tels capteurs permet d'assurer une gestion rigoureuse des cycles de lavage. En particulier, dans une machine à laver selon l'invention, l'unité de commande reçoit et exploite des informations relatives à la position de la porte, à l'état d'un cycle de lavage et au contenu de la cuve de lavage et assure ainsi des fonctionnalités robustes de commande et de contrôle.

Avantageusement et selon l'invention, ladite unité de commande est adaptée pour :
- commander le verrouillage desdits moyens de verrouillage si au moins ledit capteur de porte détecte une porte fermée, ledit capteur de cuve détecte des instruments dans ladite cuve et ledit témoin de lavage indique ledit état à laver,
- commander le déverrouillage desdits moyens de verrouillage dans les autres cas.

Une telle commande de verrouillage de porte conditionnée à la conjonction d'au moins trois conditions - porte fermée, instruments dans la cuve et témoin de lavage dans l'état à laver - permet d'une part de garantir, en situation normale de fonctionnement, que la porte ne peut être ouverte au cours d'un cycle de lavage ou tant qu'un cycle de lavage n'a pas été effectué, et permet d'autre part d'autoriser une ouverture de la porte dans tous les autres cas. En particulier, si le cycle de lavage est dans l'état lavé et que des instruments sont présents dans la cuve, il est alors possible d'ouvrir et de fermer la porte sans occasionner le verrouillage de la porte. En revanche, dès que les instruments sont déchargés de la machine, le capteur de cuve indique une absence d'instruments.

Aussi, avantageusement et selon l'invention, ladite unité de commande est adaptée pour changer l'état dudit témoin de lavage de l'état lavé à l'état à laver dès que ledit capteur de cuve ne détecte plus d'instruments dans ladite cuve de lavage. Par ailleurs, l'unité de commande est aussi adaptée pour autoriser l'interruption d'un cycle de lavage en cours, avant la fin de ce cycle de lavage, et pour n'autoriser alors l'ouverture de la porte que du côté de chargement.

Dès lors, une machine à laver selon l'invention réduit fortement les risques de confusion entre un instrument sale et un instrument propre.

Une unité de commande selon l'invention associée à des capteurs selon l'invention permet d'éviter le croisement des instruments propres avec les instruments sales.

Dans le cas d'une machine à plusieurs cuves de lavage, la même unité de commande peut être adaptée pour contrôler le fonctionnement des différentes cuves, de façon synchrone ou asynchrone.

Avantageusement et selon l'invention, lesdits moyens de verrouillage comprennent trois états, un état verrouillé dans lequel ils sont adaptés pour verrouiller ladite porte en position de lavage, un premier état déverrouillé, dit état déverrouillé de chargement dans lequel ils sont adaptés pour autoriser une ouverture de chargement de ladite porte, et un deuxième état déverrouillé, dit état déverrouillé de déchargement, dans lequel ils sont adaptés pour autoriser une ouverture de déchargement de ladite porte.

Avantageusement et selon l'invention, ladite unité de commande est adaptée pour :
- commander le passage des moyens de verrouillage dans l'état déverrouillé de chargement si au moins ledit capteur de présence détecte une absence d'instruments de lavage et un capteur de porte détecte une porte fermée,
- commander le passage des moyens de verrouillage dans l'état déverrouillé de déchargement si au moins ledit témoin de lavage indique ledit état lavé et un capteur de porte détecte une porte fermée.

Ainsi, si le capteur de porte détecte une porte fermée, l'unité de commande associée est adaptée pour commander le passage des moyens de verrouillage dans l'état déverrouillé de chargement si aucun instrument n'est présent dans la cuve de manière à pouvoir recharger la machine en instruments à laver. Cela se produit notamment lorsque, après avoir retiré des appareils propres de la machine, la porte de cette dernière est refermée. Par ailleurs, selon cette variante de l'invention, si le capteur de porte détecte une porte fermée, l'unité de commande est adaptée pour commander le passage des moyens de verrouillage dans l'état déverrouillé de déchargement si le témoin de lavage indique l'état lavé, de manière à pouvoir retirer les instruments lavés et les utiliser.

En revanche, tant que la porte est ouverte, du côté du chargement ou du côté du déchargement, l'état des moyens de verrouillage est immuable. A partir d'une position ouverte, de chargement ou de déchargement, seule une détection de la porte fermée peut autoriser un changement d'état des moyens de verrouillage.

Avantageusement, une machine à laver selon l'invention comprend une pédale, dite pédale de déchargement, agencée du côté de déchargement de la machine et adaptée pour solliciter une ouverture de déchargement de la porte de la machine, et une pédale, dite pédale de chargement, agencée du côté de chargement de la machine et adaptée pour solliciter une ouverture de chargement de la porte de la machine.

Les pédales selon l'invention sollicitent l'unité de commande. Dès lors, un utilisateur peut actionner une pédale pour solliciter l'ouverture de la porte de la machine du côté de chargement ou de déchargement. L'unité de commande détermine alors en fonction de l'état des capteurs et du témoin de lavage, si l'ouverture sollicitée est possible. Si l'ouverture sollicitée est possible, la pédale est adaptée pour entraîner le déverrouillage de la porte autorisant une ouverture de la porte du côté sollicité. Si l'ouverture sollicitée n'est pas possible, l'actionnement de la pédale est sans effet.

Dès lors, des pédales selon l'invention permettent non seulement de solliciter une ouverture de chargement ou de déchargement, mais également d'assurer le déverrouillage des moyens de verrouillage si l'ouverture sollicitée est autorisée par les moyens de verrouillage.

L'articulation bilatérale selon l'invention peut-être de tout type connu. Elle peut être réalisée par tout type de charnières bilatérales connues.

Néanmoins, avantageusement une machine selon l'invention comprend un bâti agencé autour de ladite cuve et comprenant une ouverture en regard de ladite ouverture de ladite cuve.

Selon cette variante et en combinaison, ladite porte comprend des paumelles agencées latéralement du côté de chargement et du côté de déchargement de ladite porte de la machine à laver.

Selon cette variante et en combinaison, lesdits moyens de verrouillage comprennent au moins deux pinces portées par le bâti et agencées, lorsque la porte est en position fermée, respectivement en regard d'une paumelle de la porte agencée sur le bord de chargement de la porte et en regard d'une paumelle de la porte agencée sur le bord de déchargement de la porte, chaque pince étant adaptée pour :
- dans une position, dite position verrouillée, correspondant audit état verrouillé, bloquer la paumelle de la porte agencée en regard de manière à interdire toute ouverture de la porte,
- dans une position, dite position libérée, correspondant à un état déverrouillé, permettre une libération de ladite paumelle de la porte agencée du côté de cette pince de manière à autoriser une ouverture latérale de la porte du côté de cette pince.

Le bâti, les pinces et les paumelles de porte permettent de former l'articulation bilatérale de la porte.

Une telle articulation bilatérale peut comprendre un nombre varié de pinces.

Selon une variante de l'invention, lesdits moyens de verrouillage comprennent deux séries de pinces portées par le bâti, une première série de pinces agencées, lorsque que la porte est en position fermée, en regard d'une série de paumelles de la porte agencées sur le bord de chargement de la porte ; et une deuxième série de pinces agencées, lorsque la porte est en position fermée, en regard d'une série de paumelles de la porte agencées sur le bord de déchargement de la porte.

De préférence, chaque série de pinces est formée d'une paire de pinces.

Le bâti, les pinces et les paumelles de porte permettent de former l'articulation bilatérale de la porte. Les pinces peuvent présenter différentes formes.

Néanmoins, avantageusement et selon l'invention, chaque pince comprend deux leviers munis respectivement de deux crochets conjugués adaptés pour pouvoir former une pince de saisie d'une paumelle de ladite porte et pour pouvoir être écartés l'un de l'autre de manière à autoriser la libération de cette paumelle de ladite porte et permettre une ouverture latérale de la porte.

Selon cette variante, chaque pince comprend deux leviers articulés munis de deux crochets. Ces deux leviers sont adaptés pour pouvoir être déplacés l'un par rapport à l'autre de manière à pouvoir former, selon la position du premier levier par rapport au second, soit une pince de saisie d'une paumelle de porte de manière à assurer un verrouillage de la porte, soit une ouverture de la pince pour la libération d'une paumelle de porte. Dès lors, chaque pince ainsi formée peut être dans une première configuration correspondant à ladite position libérée et dans une deuxième configuration correspondant à ladite première position verrouillée.

Le changement de configuration de chacune des pinces peut être assuré par tout dispositif connu adapté pour déplacer les leviers l'un par rapport à l'autre. Ces déplacements peuvent être le résultat d'une action manuelle développée par un opérateur ou être obtenus pas des mécanismes commandés électriques ou mécaniques.

Néanmoins, avantageusement et selon l'invention, chaque pince comprend deux leviers montés sur le bâti par l'intermédiaire d'au moins deux tourillons fixes par rapport au bâti, dit tourillons de guidage et d'au moins un coulisseau mobile par rapport au bâti, dit coulisseau de déplacement, ledit coulisseau de déplacement étant adapté pour entraîner le déplacement des leviers de cette pince dans un plan orthogonal audit plan d'accès entre une position rétractée dans laquelle les leviers maintiennent la porte en position de lavage et au moins une position déployée dans laquelle les leviers autorisent une ouverture de la porte, la porte étant écartée de la cuve de lavage.

Avantageusement et selon l'invention, chaque levier s'étend dans un plan perpendiculaire au plan d'accès et comprend une lumière oblongue, dite lumière de déplacement, s'étendant selon une direction principale parallèle audit plan d'accès et à travers laquelle est agencée ledit coulisseau de déplacement, et deux lumières oblongues, dite lumières de guidage, s'étendant selon une direction principale orthogonale audit plan d'accès, agencées de part et d'autre de ladite lumière de déplacement et à travers lesquelles sont agencées lesdites tourillons de guidages fixés par rapport au bâti.

Avantageusement et selon l'invention, ladite articulation de ladite porte comprend, sur chaque côté de chargement et de déchargement, un mécanisme, dit mécanisme excentrique, comprenant un arbre parallèle audit plan d'accès adapté pour être entraîné en rotation par des moyens d'entraînement et à chaque extrémité duquel sont agencés deux coulisseaux de déplacement excentrés s'étendant selon une direction parallèle à la direction de l'arbre, chaque coulisseau traversant lesdites lumières oblongues de déplacement desdits leviers formant une pince de sorte que la rotation excentrée desdits coulisseau force un déplacement desdites pinces orthogonalement audit plan d'accès.

Un mécanisme excentrique selon l'invention permet d'assurer un changement de position des pinces de l'articulation de la machine selon l'invention. En particulier, un mécanisme excentrique selon l'invention comprenant un arbre aux extrémités duquel sont agencés des coulisseaux de déplacement excentrés, chaque coulisseau étant adapté pour être logé dans une lumière de déplacement, permet de transformer un mouvement rotatif de l'arbre en un mouvement de translation des pinces selon une direction perpendiculaire aux lumières oblongues.

Selon cette variante, en combinaison et selon l'invention, l'une des lumières oblongues de guidage d'un des leviers, dit levier articulé, présente une portion inférieure inclinée par rapport à la lumière de guidage du levier en regard de telle sorte que les portions inférieures de ces lumières de guidage sont non superposées.

Dès lors, un déplacement de ces leviers vers la position totalement déployée, résultant du déplacement du coulisseau excentrique de déplacement, entraîne un écartement entre les deux crochets des deux leviers du fait d'un contact mécanique entre le tourillon de guidage fixe passant à travers cette portion des lumières de guidages non superposées, ce qui permet la libération d'une paumelle de la porte et autorise une ouverture de la porte. La pince est alors dans ladite position libérée.

La combinaison des leviers articulés, d'une lumière oblongue de déplacement, de lumières oblongues de guidage, de tourillons de guidage et d'un mécanisme excentrique, permet de réaliser une pince adaptée pour :
- dans la position rétractée, correspondant à ladite position verrouillée, bloquer la paumelle de la porte agencée en regard de cette pince,
- dans une position totalement déployée, correspondant à ladite position libérée, permettre une libération de ladite paumelle de la porte agencée du côté de cette pince de manière à permettre une ouverture latérale de la porte du côté de cette pince.

Avantageusement et selon l'invention, le coulisseau de déplacement est adapté pour positionner chaque pince, dans une position intermédiaire déployée, dite position de pivot, entre la position rétractée et la position totalement déployée, et dans laquelle elle forme un gond de pivotement de ladite paumelle de la porte agencée du côté de la pince de manière à permettre une ouverture latérale de la porte du côté opposé.

Chaque pince étant adaptée pour former un gond de pivotement, la série de pinces agencée du côté de chargement de la machine est adaptée pour former un axe de pivotement de la porte pour permettre une ouverture de la porte du côté de déchargement, et la série de pince agencée du côté de déchargement de la machine est adaptée pour former un axe de pivotement de la porte pour permettre une ouverture de la porte du côté de chargement.

Dans cette position de pivot, la porte est maintenue écartée au dessus de la cuve par l'intermédiaire des pinces. La porte n'étant plus en contact étroit et étanche avec la cuve, elle est susceptible de pivoter autour de l'un des axes de pivots formés par les pinces sans risquer d'endommager la cuve.

L'agencement des pinces sur le bâti est de préférence déterminé de manière à ce que les deux axes de pivotement de chargement et de déchargement ainsi réalisés soient parallèles l'un à l'autre, d'un côté et de l'autre de la cuve.

Dès lors, selon cette variante de l'invention, le coulisseau de déplacement est adapté pour positionner chaque pince :
- dans ladite position rétractée dans laquelle est bloque la paumelle de la porte agencée en regard de cette pince,
- dans ladite position de pivot dans laquelle elle forme un gond de pivotement de ladite paumelle de la porte agencée du côté de cette pince de manière à faciliter l'ouverture latérale de la porte du côté opposé,
- dans ladite position totalement déployée dans laquelle elle autorise une libération de ladite paumelle de la porte agencée du côté de cette pince de manière à permettre une ouverture latérale de la porte du côté de cette pince.

Selon l'invention, la position du coulisseau de déplacement excentré conditionne la position des pinces. Selon un mode de réalisation de l'invention, le mécanisme excentrique est adapté pour assurer que la pince passe de la position de verrouillage, c'est-à-dire de la position rétractée, à la position de pivot, c'est-à-dire à la position intermédiaire, lorsque le coulisseau de déplacement excentré a effectué une rotation de 130°. De même, le mécanisme excentrique est adapté pour assurer que la pince passe de la position de pivot, c'est-à-dire de la position intermédiaire, à la position déverrouillée, c'est-à-dire à la position totalement déployée, lorsque le coulisseau de déplacement excentré a effectué une rotation de 30° supplémentaire.

Les mécanismes excentriques selon l'invention peuvent être de tout type.

Néanmoins, avantageusement et selon l'invention, lesdits moyens d'entraînements en rotation desdits arbres desdits mécanismes excentriques comprennent des moteurs électriques.

Ces moteurs électriques peuvent être déclenchés par les pédales d'actionnement agencées du côté de chargement et du côté de déchargement.

L'actionnement des mécanismes excentriques par l'intermédiaire de pédales facilite les opérations de changement d'état des moyens de verrouillage, notamment par un opérateur qui porte dans ses bras des instruments médicaux et/ou chirurgicaux à laver qu'il doit installer dans la cuve de lavage.

Avantageusement, une machine selon l'invention comprend des capteurs de position de chaque pince de manière à pouvoir déterminer la position de la porte de la machine à laver.

Avantageusement, une machine selon l'invention comprend deux bras articulés télescopiques agencés entre les bords latéraux de la porte et la machine et adaptés pour pouvoir se déployer sur commande pour assurer une ouverture automatique de la porte et pour pouvoir se rétracter sur commande pour permettre une fermeture de la porte.

De préférence, l'activation des bras télescopiques est commandée par les pédales d'actionnement et conditionnée aux autorisations de l'unité de commande.

L'invention concerne en outre une machine à laver des instruments médicaux et/ou chirurgicaux caractérisée en combinaison par tout ou partie des caractéristiques mentionnées ci-dessus ou ci-après.

D'autres caractéristiques, buts et avantages de l'invention apparaîtront à la lecture de la description suivante qui présente à titre d'exemple non limitatif un mode de réalisation de l'invention, en référence aux dessins annexés ; sur ces dessins :
- la figure 1 est une vue schématique en perspective d'une machine à laver des instruments à usage médical et/ou chirurgical selon un mode de réalisation de l'invention,
- les figures 2a et 2b sont des vues schématiques en perspective d'une machine à laver des instruments à usage médical et/ou chirurgical équipée d'une porte bilatérale selon un mode de réalisation de l'invention représentée respectivement dans une position d'ouverture de chargement d'instruments à laver et dans une position d'ouverture de déchargement d'instruments lavés,
- la figure 3 est une vue schématique en perspective d'un bâti d'une articulation d'une porte d'une machine à laver des instruments à usage médical selon un mode de réalisation de l'invention,
- la figure 4 est une vue schématique en perspective éclatée d'une pince de verrouillage d'une porte d'une machine à laver des appareils à usage médical selon un mode de réalisation de l'invention,
- les figures 5a, 5b et 5c sont des vues schématiques de côté des différentes positions prises par des pinces de verrouillage d'une porte d'une machine à laver des instruments à usage médical et/ou chirurgical selon un mode de réalisation de l'invention,
- la figure 6 est une vue schématique en coupe d'un mécanisme excentrique d'entraînement d'une pince de verrouillage d'une machine à laver des instruments à usage médical et/ou chirurgical selon un mode de réalisation de l'invention.

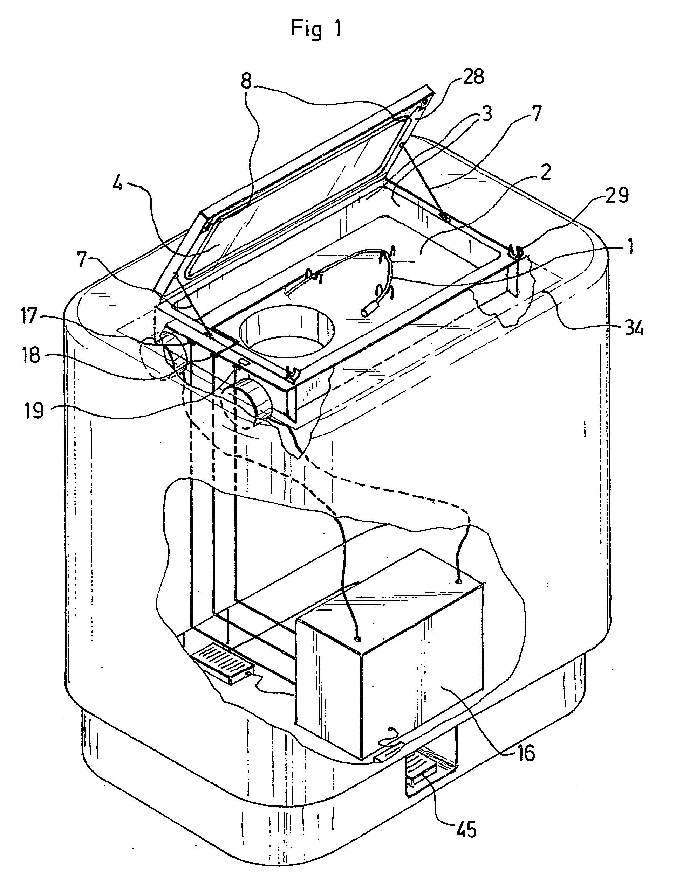

La figure 1 est une vue schématique d'une machine à laver des instruments 1 à usage médical et/ou chirurgical comprenant une cuve 2 de lavage délimitée par des parois 3 et comprenant une ouverture d'accès à la cuve 2 de lavage qui s'étend dans un plan, dit plan 34 d'accès.

Selon le mode de réalisation des figures, le plan 34 d'accès est horizontal. Néanmoins, selon d'autres variantes de réalisation, le plan d'accès 34 peut être vertical, voir oblique.

Les instruments 1 à usage médical et/ou chirurgical sont typiquement des fibroscopes, des endoscopes, des sondes d'échocardiographie, des sondes d'échographie, et tout autre instrument, notamment de type thermolabile, qui requièrent un lavage minutieux avant chaque utilisation sur un patient.

Une machine à laver selon l'invention comprend une porte 4 montée en regard de l'ouverture de la cuve 2 de lavage. Cette porte 4 est adaptée pour passer d'une position, dite position de lavage, dans laquelle elle recouvre de façon étanche ladite ouverture de ladite cuve 2 de lavage de manière à permettre un cycle de lavage à au moins une position, dite position ouverte, dans laquelle elle permet un accès à la cuve 2 de lavage pour charger des instruments 1 à laver et/ou décharger des instruments 1 lavés. La figure 1 présente la porte 4 en position ouverte.

Comme mentionné précédemment, selon le mode de réalisation des figures, le plan 34 d'accès est horizontal, de telle sorte qu'en position fermée, la porte 4 s'étend dans un plan horizontal. Rien n'empêche néanmoins d'envisager d'autres configurations, notamment une configuration dans laquelle le plan 34 d'accès est vertical, de telle sorte que la porte 4 s'étendrait en position fermée dans un plan vertical.

Selon un mode de réalisation particulièrement avantageux, la porte 4, la cuve 2 et la machine sont réalisées de telle manière qu'en position fermée la porte 4 soit dans le même plan que le carter extérieur de la machine, c'est-à-dire, qu'une fois la porte fermée, aucun élément ne fasse saillie de la machine, de telle sorte qu'un opérateur puisse aisément déceler en un coup d'oeil une porte bien fermée verrouillée en position de lavage et une porte non correctement fermée.

L'étanchéité entre la cuve 2 et la porte 4 peut être obtenue par tous les moyens connus. Cette étanchéité est par exemple obtenue par des joints d'étanchéité, par exemple en caoutchouc ou en résine de silicone, agencés sur la périphérie de la face de la porte en regard de la cuve 2 en position fermée, dite face 28 intérieure, de telle sorte que lorsque la porte 4 est en position fermée, ces joints en caoutchouc ou en résine de silicone puissent être écrasés entre la porte 4 et les bords supérieurs de la cuve 2.

Une machine selon l'invention comprend des moyens de verrouillage à au moins deux états de ladite porte 4, un état verrouillé dans lequel lesdits moyens de verrouillage sont adaptés pour verrouiller ladite porte 4 en position de lavage de telle sorte qu'il soit impossible d'ouvrir ladite porte 4 au cours d'un cycle de lavage, et un état déverrouillé dans lequel lesdits moyens de verrouillage sont adaptés pour autoriser une ouverture de ladite porte 4.

Ces moyens de verrouillage peuvent être de tout type. Ils peuvent être formés par des systèmes à serrure, à verrou, à loquet, etc.

Selon le mode de réalisation de la figure 1, ces moyens de verrouillage sont formés par des pinces 29 commandées adaptées pour pouvoir enserrer des paumelles 8 agencées sur ladite face 28 intérieure de la porte 4 et pour pouvoir libércr ces paumelles 8.

Selon le mode de réalisation des figures, chaque paumelle 8 est formée par un cylindre métallique s'étendant selon une direction parallèle au plan de la porte 4, sur une longueur de l'ordre de 1 cm, relié à la face 28 intérieure de la porte 4 par des entretoises agencées à chacune des extrémités du cylindre ou fixé à la face 28 intérieure par brasure ou soudure entre la face 28 intérieure et le cylindre, les extrémités, du cylindre présentant pour ce faire un diamètre plus grand que le diamètre de la partie centrale du cylindre destinée à être enserrée par une paumelle 8. Chaque paumelle 8 est agencée sur la porte 4 de manière à ce qu'elle soit en regard d'une pince 29 lorsque la porte 4 est fermée.

Une machine à laver selon l'invention comprend, associée aux moyens de verrouillage, une unité 16 de commande adaptée pour commander les moyens de verrouillage. Cette unité 16 de commande peut être associée à un automate programmable réalisé par des moyens électriques et/ou informatiques et/ou pneumatiques et/ou hydrauliques, etc. En particulier, un tel automate peut être équipé d'un ordinateur personnel, comprendre un microprocesseur, une interface de commande, un progiciel, etc.

Une unité 16 de commande selon l'invention est avantageusement associée à au moins un capteur de position de ladite porte, dit capteur 17 de porte, adapté pour détecter une position ouverte de ladite porte 4 et une position fermée de ladite porte, au moins un capteur de présence d'instruments médicaux et/ou chirurgicaux dans ladite cuve, dit capteur 18 de cuve, et au moins un témoin 19 de lavage à deux états, un état lavé correspondant à une fin de cycle de lavage, et un état à laver, correspondant à un cycle de lavage à faire.

La figure 1 présente de façon schématique les connexions entre les différents capteurs et témoin, et l'unité 16 de commande.

Un capteur 17 de porte selon l'invention et un capteur 18 de cuve selon l'invention peuvent être des capteurs à contacts, des capteurs optiques, des capteurs magnétiques, etc.

Avantageusement, un capteur 18 de cuve selon l'invention est associé à des moyens de reconnaissance des instruments 1 logés dans la cuve 2. Ces moyens de reconnaissance peuvent être de tout type connu en soi. Ils comprennent de préférence des moyens de lecture (optique et/ou magnétique et/ou électrique et/ou radiofréquence et/ou mécanique) d'informations portées par chaque instrument. Ces moyens de reconnaissance sont par exemple des moyens de lecture optique adaptés pour lire une empreinte agencée sur un instrument 1 médical et/ou chirurgical chargé dans la cuve, de manière à ce que ce capteur puisse transmettre à l'unité de commande 16, une information relative au type d'instrument chargé dans la machine. Cette empreinte comprend par exemple un code à barres et les moyens de lecture optique comprennent des moyens connus de lecture d'un code à barres.

Selon un autre mode de réalisation de l'invention, les moyens de reconnaissance des instruments 1 logés dans la cuve 2 associés au capteur 18 de cuve comprennent des moyens de lecture à distance de puces électroniques radiofréquences (RFID) agencées sur un instrument 1 logé dans la cuve 2 et adaptées pour mémoriser des informations relatives à cet instrument, et transmettre ces informations aux moyens de lecture. De tels moyens permettent une reconnaissance automatique et systématique de l'instrument sans aucune action spécifique.

Un tel capteur 18 de cuve permet par exemple d'adapter le type de lavage au type d'instrument logé dans la machine reconnu par les moyens de reconnaissance.

Selon un mode avantageux de réalisation de l'invention, l'unité 16 de commande est adaptée pour commander le verrouillage desdits moyens de verrouillage si, au moins simultanément, ledit capteur 17 de porte détecte une porte 4 fermée, ledit capteur 18 de cuve détecte des instruments 1 dans ladite cuve 2 de lavage et ledit témoin 19 de lavage indique ledit état à laver.

De plus, l'unité 16 de commande est avantageusement adaptée pour changer l'état dudit témoin 19 de lavage de l'état lavé à l'état à laver si ledit capteur 18 de cuve ne détecte plus d'instruments 1 dans ladite cuve 2 de lavage.

Un témoin 19 de lavage selon l'invention peut comprendre un témoin numérique associé à une mémoire centrale standard que l'unité 16 de commande interroge pour déterminer l'état de ce dernier et modifie pour changer l'état de ce dernier. Ce témoin 19 peut également comprendre un témoin lumineux visible de l'extérieur de la machine de manière à ce qu'un opérateur puisse déterminer l'état de ce témoin.

Une machine à laver selon l'invention comprend également un circuit d'alimentation de la machine en compositions de lavage débouchant dans la cuve de lavage. Ce circuit, connu en lui-même, n'est pas représenté sur les figures à des fins de clarté.

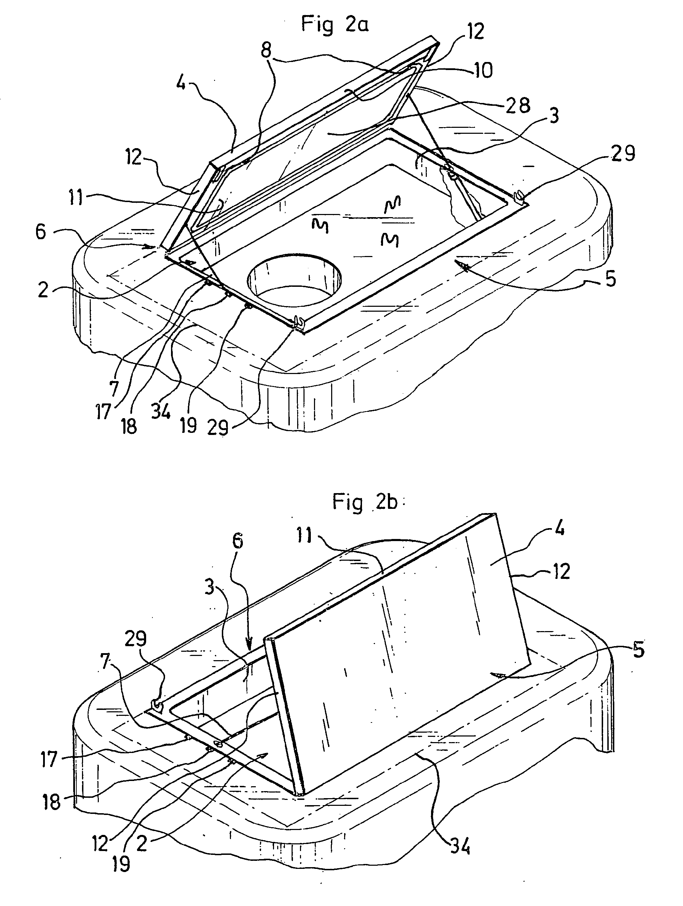

Comme représenté sur les figures 2a et 2b, la porte 4 est montée sur une articulation adaptée pour permettre deux ouvertures distinctes.

A noter que sur les figures 2a, 2b et 1, les pinces formant l'axe de pivotement de la porte ne sont pas représentées à des fins de clarté.

La porte 4 est montée sur une articulation, dite articulation bilatérale, adaptée pour d'une part permettre une première ouverture latérale de la porte 4 sur un premier côté, dit côté 5 de chargement de la cuve - notamment de la machine -, et d'autre part, permettre une seconde ouverture latérale de la porte sur le côté opposé, dit côté 6 de déchargement de la cuve - notamment de la machine à laver-.

La figure 2a est une vue de la machine équipée d'une porte 4 montée sur une articulation bilatérale et ouverte sur le côté 5 de chargement de manière à permettre le chargement d'instruments 1 dans la machine et la figure 2b est une vue de la même machine dont la porte 4 est ouverte sur le côté 6 de déchargement de manière à permettre le déchargement d'instruments 1 lavés.

Le passage de la porte 4 de la position fermée à la position ouverte du côté 6 de déchargement n'est possible que si le témoin 19 de lavage indique ledit état lavé.

Le témoin 19 de lavage passe de l'état à laver à l'état lavé, dès qu'un cycle de lavage se termine de façon normale. Les différentes étapes d'un cycle de lavage et les moyens de contrôle de l'exécution de chacune des étapes ne sont pas décrites et sont connues de la plupart des machines à laver.

Selon l'invention, si un cycle ne s'est pas correctement déroulé, ou si un opérateur décide de suspendre l'exécution d'un cycle, la machine à laver ne peut être ouverte que du côté de chargement de la machine.

Selon un mode de réalisation de l'invention, la machine est en outre équipée d'un système de lecture d'une identification d'un utilisateur adaptée pour autoriser ou non la mise en route ou l'arrêt de la machine. Cette identification peut être réalisée par tous moyens connus, notamment par une carte magnétique, par une carte à puce avec ou sans contact (RFID), par saisie d'un code sur un clavier de la machine, par lecture optique d'un code à barres, etc.

Dans le mode de réalisation représenté sur les figures 2a et 2b, la porte 4 présente une forme parallélépipédique adaptée pour recouvrir l'ouverture d'accès de la cuve 2, qui présente, selon le mode de réalisation des figures, une ouverture globalement rectangulaire. La porte 4 est plane et est formée d'un matériau rigide. Selon d'autres modes de réalisation, la cuve 2 et la porte 4 peuvent présenter d'autres formes. La porte 4 présente des dimensions supérieures aux dimensions de l'ouverture d'accès de la cuve 2 de manière à ce qu'elle puisse couvrir intégralement l'ouverture d'accès de la cuve 2 aux fins d'autoriser un lavage des instruments 1 logés dans la cuve 2.

Selon le mode de réalisation des figures, la porte 4 est délimitée par un bord 10 de chargement, un bord 11 de déchargement opposé et parallèle au bord 10 de chargement, et deux bords, dits bords 12 latéraux, orthogonaux au bord 10 de chargement et au bord 11 de déchargement. Le bord 10 de chargement est un bord adapté pour être articulé du côté 5 de chargement de la machine et le bord 11 de déchargement est un :bord adapté pour être articulé du côté 6 de déchargement de la machine.

Selon l'invention, chaque bord 10 de chargement et 11 de déchargement de la porte 4 est équipé de deux paumelles 8.

Chaque paumelle 8 est adaptée pour être enserrée par une pince 29 décrite ci-après. Ces pinces 29 forment les moyens de verrouillage de la porte 4.

Selon le mode de réalisation, décrit ci-après, les paumelles 8 d'un même bord s'étendent selon une même direction, parallèle à la direction dans laquelle s'étendent les paumelles 8 du bord opposé et parallèle au plan d'accès 34, une fois la porte 4 en position fermée.

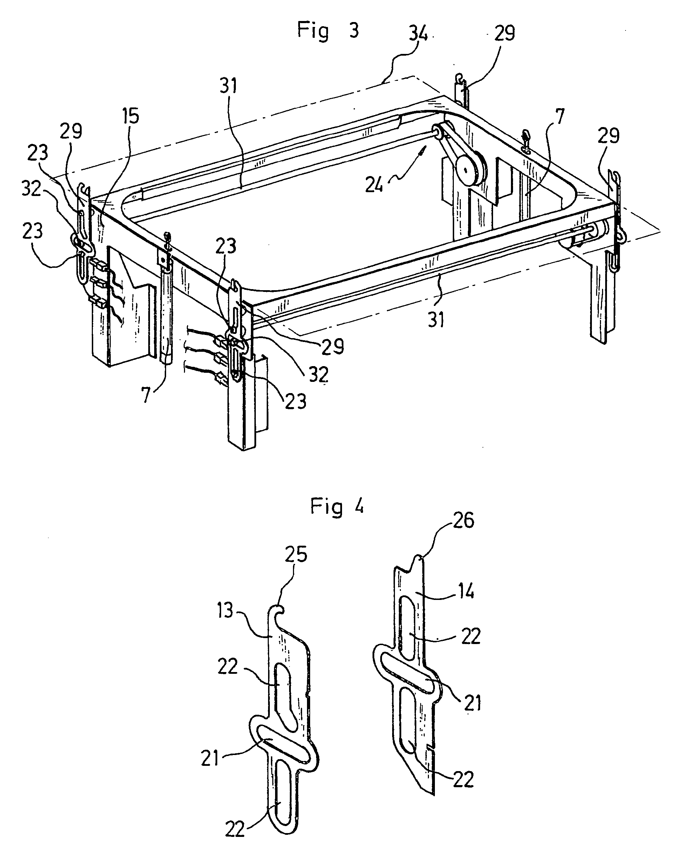

Ces pinces 29 sont fixées sur un bâti 15, tel que représenté sur la figure 3. Le bâti 15 de l'articulation est agencé autour de la cuve 2 et comprend des parois latérales et une ouverture en regard de l'ouverture de la cuve 2.

Selon le mode de réalisation des figures, deux paires de pinces 29 sont portées par le bâti 15 et sont agencées de façon à être, lorsque que la porte 4 est en position fermée, respectivement en regard d'une paire de paumelles 8 de la porte 4 agencée sur le bord 10 de chargement et d'une paire de paumelles 8 de la porte agencée sur le bord 11 de déchargement de la porte.

La paire de pinces 29 agencée du côté 10 de chargement de la machine est adaptée pour former, lorsque que chaque pince 29 de cette paire de pinces 29 est dans la position de pivot, un axe de pivotement de la porte pour permettre une ouverture de la porte 4 du côté 6 de déchargement, et la paire de pinces 29 agencée du côté 6 de déchargement de la machine est adaptée pour former un axe de pivotement de la porte 4 lors d'une ouverture de la porte 4 du côté 5 de chargement.

Ces axes de pivotement sont parallèles entre eux et parallèle au plan d'accès 34 et sont matérialisés sur les figures par les arbres 31.

Chaque pince 29 est adaptée pour, dans une première position, dite position verrouillée, bloquer la paumelle 8 de la porte 4 agencée en regard ; dans une deuxième position, dite position libérée, permettre une libération de ladite paumelle 8 de la porte 4 agencée du côté de cette pince 29 de manière à permettre une ouverture latérale de la porte 4 du côté de cette pince ; et dans une troisième position, dite position de pivot, former un gond de pivotement de ladite paumelle 8 de la porte 4 agencée du côté de la pince de manière à permettre une ouverture latérale de la porte 4 du côté opposé.

Pour ce faire, une pince 29 selon l'invention comprend, tel que représenté sur la figure 4, deux leviers 13, 14 conjugués. Le levier 13 comprend, à une de ses extrémités, un crochet 25 adapté pour former en coopération avec un crochet 26 agencé à une extrémité du levier 14 conjugué, une pince de maintien d'une paumelle 8 d'une porte 4 lorsque les deux leviers 13, 14 sont superposés l'un à l'autre. Le pivotement d'un levier par rapport à l'autre permet un écartement des crochets l'un de l'autre et une libération d'une paumelle 8 bloquée entre les crochets.

Ce pivotement d'un levier par rapport à l'autre est réalisé par la combinaison des structures des leviers décrites ci-après et d'au moins un mécanisme, dit mécanisme excentrique, décrit ci-après.

Chaque levier est en forme générale de plaquette s'étendant orthogonalement au plan 34 d'accès et à l'axe de pivotement formé par chaque paire de pinces 29.

Chaque levier comprend une lumière oblongue, dite lumière 21 de déplacement, s'étendant selon une direction parallèle audit plan 34 d'accès, une fois la pince 29 montée sur le bâti 15.

Chaque levier comprend également deux lumières oblongues, dites lumières 22 de guidage, perpendiculaires à la lumière 21 de déplacement. De préférence, les lumières de guidage de chaque levier sont agencées de part et d'autre de la lumière 21 de déplacement.

Chaque lumière 21 de déplacement de chaque levier permet de déplacer ce levier perpendiculairement à cette lumière 21 de déplacement.

Pour ce faire, le bâti 15 porte des mécanismes, dits mécanismes 24 excentriques, adaptés pour assurer ce déplacement des leviers.

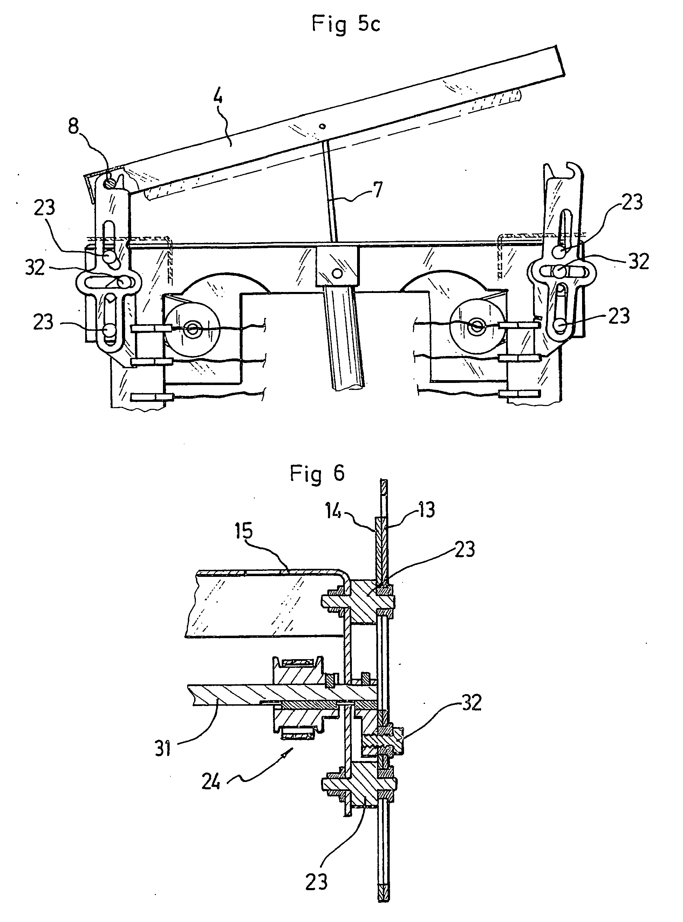

Chaque mécanisme 24 excentrique comprend, tel que représenté notamment sur la figure 6, un arbre 31, s'étendant parallèlement audit plan 34 d'accès et à chaque extrémité duquel est agencé un coulisseau 32 excentré par rapport à l'arbre 31 et parallèle à l'arbre 31.

Chaque arbre 31 est relié à des moyens d'entraînement en rotation de cet arbre 31. Ces moyens d'entraînement en rotation peuvent être de tout type, mécanique, électrique, hydraulique, etc. Selon le mode de réalisation des figures, ces moyens d'entraînement en rotation sont associés à des moteurs électriques.

La mise en rotation d'un arbre 31 entraîne la rotation des coulisseaux 32 excentrés. Chaque coulisseau 32 excentré passe à travers les lumières 21 de déplacement des leviers 13, 14 formant une pince 29.

Par ailleurs, le bâti 15 comprend des tourillons 23 solidaires du bâti et adaptés pour se loger dans les lumières 22 de guidage des leviers 13, 14.

Dès lors, la rotation d'un coulisseau 32 excentré autour de l'arbre 31 contraint et entraîne le déplacement de chaque levier suivant la direction des lumières de guidage.

La longueur d'au moins une des lumières 22 de guidage est choisie de manière à ce que lors du déplacement des leviers 13, 14 perpendiculairement audit plan 34 d'accès, au moins un tourillon 23 soit en contact mécanique avec l'extrémité inférieure d'une lumière 22 de guidage.

Ainsi, selon l'invention, cette lumière 22 de guidage de l'un des leviers, dit levier 13 articulé, présente une portion inférieure inclinée par rapport à la direction de la lumière de guidage du levier 14 superposé. De telle manière, lors du déplacement des leviers 13, 14, guidé par ce tourillon 23 de guidage, la fin de course de ce tourillon 23 de guidage dans les lumières de guidage non superposées provoque un pivotement du levier 13 articulé par rapport au levier 14. Ce pivotement entraîne un écartement l'un de l'autre des deux crochets 25, 26 des deux leviers 13, 14 et permet la libération d'une paumelle 8 de la porte hors de la pince 29, ladite pince 29 est alors dans la position libérée.

La combinaison des tourillons 23 de guidage, des lumières 22 de guidage, des mécanismes 24 excentriques, des lumières 21 de déplacement et des leviers 13, 14, permet de réaliser des moyens de verrouillage d'une porte adaptés pour, dans une première position, bloquer la paumelle 8 d'une porte 4 et dans une deuxième position, permettre une libération d'une paumelle 8 de porte 4.

Par ailleurs, la combinaison des leviers 13, 14 articulés, d'une lumière 21 oblongue de déplacement, de lumières 22 oblongues de guidage, de tourillons 23 de guidage et d'un mécanisme 24 excentrique, permet de réaliser une pince 29 adaptée pour :
- dans une position rétractée, correspondant à ladite position verrouillée, bloquer la paumelle 8 de la porte 4 agencée en regard de cette pince 29,
- dans une position totalement déployée, correspondant à ladite position libérée, permettre une libération de ladite paumelle 8 de la porte 4 agencée du côté de cette pince de manière à permettre une ouverture latérale de la porte 4 du côté de cette pince 29.

Au cours de la course des lumières de guidage autour du tourillon fixe de guidage, les crochets des leviers 13, 14 restent fermés tant qu'il n'y a pas de contact entre le tourillon de guidage et la portion inclinée des lumières.

Dès lors, de façon avantageuse, cette position est repérée, par exemple par des capteurs de position, comme étant une position, dite position de pivot.

Dans cette position de pivot, la porte 4 est maintenue en suspension au-dessus de la cuve 2 par l'intermédiaire des pinces 29. La porte n'étant plus en contact étroit et étanche avec la cuve, elle est susceptible de pivoter autour de l'un des axes de pivots formés par les pinces 29 sans risquer d'endommager la cuve.

Dès lors, le déplacement du coulisseau 32 de déplacement est adapté pour positionner chaque pince 29, dans une position rétractée dans laquelle les deux leviers 13, 14 forment une pince de maintien de la paumelle 8 agencée en regard ; dans une position dite de pivot, dans laquelle les leviers forment un gond de pivotement d'une porte, la porte étant surélevée par rapport à sa position de lavage ; une position totalement déployée dans laquelle les leviers 13, 14 sont pivotés l'un par rapport à l'autre de telle sorte que les crochets 25, 26 agencés à leurs extrémités sont écartés l'un de l'autre et permettent la libération d'une paumelle de porte hors de la pince 29.

L'actionnement des mécanismes 24 excentriques entraîne le déplacement des leviers et détermine donc la position des pinces 29 formant les moyens de verrouillage.

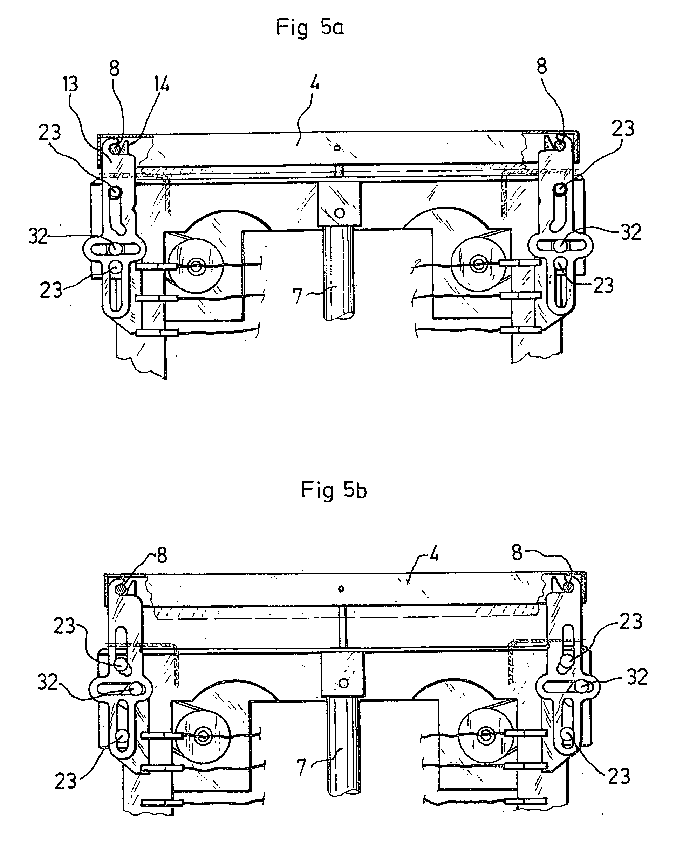

La première position, dite position verrouillée, représentée sur la figure 5a, correspond à une position du mécanisme 24 excentrique dans laquelle le coulisseau 32 excentré maintien la pince 29 en position rétractée.

La deuxième position, dite position libérée, représentée sur la figure 5c, correspond à une position du mécanisme 24 excentrique dans laquelle le coulisseau 32 excentré a effectué une rotation de 160 degrés par rapport à la première position autour de l'arbre 31 et amène la pince 29 en position totalement déployée.

La troisième position, dite position de pivot, représentée sur la figure 5b, correspond à une position du mécanisme 24 excentrique dans laquelle le coulisseau 32 excentré a effectué une rotation de l'ordre de 130 degrés par rapport à le première position autour de l'arbre 31 amène les leviers dans une position intermédiaire entre la position rétractée et la position totalement déployée.

Ainsi, en situation normale de fonctionnement, l'actionnement du mécanisme excentrique permet le passage de la position verrouillée à la position de pivot, puis à la position libérée.

Selon un mode de réalisation avantageux de l'invention, les moyens d'entraînement en rotation des arbres excentriques sont actionnés par des pédales. La machine comprend alors une pédale, dite pédale de déchargement, agencée du côté 6 de déchargement de la machine et adaptée pour solliciter une ouverture de déchargement de la porte et une pédale, dite pédale 45 de chargement, agencée du côté 5 de chargement de la machine et adaptée pour solliciter une ouverture de chargement de la porte de la machine.

Dans le cas d'une machine à plusieurs cuves, de préférence, la machine comprend une pédale de chargement par cuve et une pédale de déchargement par cuve. Néanmoins, dans le cas d'une machine à plusieurs cuves à fonctionnement synchrone, la machine peut comprendre une unique pédale de déchargement et une unique pédale de chargement.

L'unité 16 de commande détermine alors si l'action sollicitée peut être satisfaite selon l'état des capteurs de la machine.

Selon ce de mode de réalisation, l'unité 16 de commande autorise une ouverture de chargement de la machine si aucun cycle de lavage n'est en cours, si le capteur 18 de cuve indique que la cuve 2 est vide, et si la pédale 45 de chargement de la machine est actionnée.

Selon ce mode de réalisation, l'unité 16 de commande autorise une ouverture de déchargement si le témoin 19 de lavage indique un état lavé, si le capteur 18 de cuve indique la présence d'un instrument dans la cuve 2 et si la pédale de déchargement a été actionnée.

Selon cette variante, l'identification d'un utilisateur autorisé par les moyens d'identification peut également conditionner l'ouverture de déchargement.

Selon un mode avantageux de réalisation de l'invention, les pédales de chargement et de déchargement sont également adaptées pour solliciter la fermeture de la porte. Pour ce faire, un opérateur doit actionner la pédale agencée du côté où la porte est ouverte.

Selon l'invention, l'unité de commande est adaptée pour ne permettre, à un instant donné, qu'une seule action. Cette dernière dépend, comme mentionné précédemment, de l'état des capteurs (position de la porte, instruments dans la cuve, utilisateur autorisé), du témoin de lavage et des pédales de commande.

Selon un mode avantageux de réalisation de l'invention, la porte 4 est également articulée autour de deux bras 7 articulés télescopiques agencés entre les bords 12 latéraux de la porte 4 et le bâti 15 de la machine.

Chaque bras 7 articulé forme une liaison pivot avec la porte 4. Cette liaison pivot est de préférence agencée au milieu du bord 12 latéral de manière à ce que la porte puisse pivoter autour de cette liaison pivot lors d'une ouverture de chargement et d'une ouverture de déchargement. De préférence, chaque bras 7 articulé forme également une liaison pivot avec le bâti 15. Les axes de pivotement de ces différentes liaisons pivots sont parallèles et de préférence parallèles aux axes de pivotement de la porte réalisés par les pinces 29.

Selon un mode particulièrement avantageux de réalisation de l'invention, les bras 7 articulés sont des vérins adaptés pour être déployés sur commande de l'unité 16 de commande. Le déploiement des bras 7 n'est autorisé que si les moyens de verrouillage sont dans un état déverrouillé de chargement ou de déchargement. En revanche, les bras 7 doivent être rétractés pour que les moyens de verrouillage puissent verrouiller la porte 4 en position de lavage.

De préférence, les vérins sont des vérins pneumatiques, de telle sorte que le passage de la porte de la position fermée à la position ouverte puisse s'effectuer par la mise sous pression des vérins, et que le passage de la porte de la position ouverte à la position fermée puisse être réalisé par la vidange des vérins par l'intermédiaire d'une soupape d'échappement, ce qui entraîne la fermeture de la porte par un phénomène naturel de gravitation.

Dès lors, l'unité 16 de commande pilote les moyens de verrouillage et active le déplacement des bras articulés lorsque les moyens de verrouillage sont dans l'état correspondant.

Selon un mode de réalisation avantageux combinant les différents modes décrits, le passage de la porte de la position fermée verrouillé à, par exemple, une position ouverte de déchargement, implique séquentiellement, un actionnement de la pédale agencée du côté de déchargement, une autorisation d'ouverture délivrée par l'unité de commande (qui dépend de l'état des capteurs de cuve, de porte et du témoin de lavage), une mise en route des moteurs électriques des mécanismes excentriques pour positionner les pinces dans la position correspondante et une alimentation des vérins pneumatiques.

Selon ce synopsis, toutes les pinces 29 sont initialement en position rétractée correspondant à la position verrouillée. La mise en route des moteurs électriques des mécanismes excentriques entraîne la rotation des coulisseaux 32 excentriques qui entraînent le déplacement de toutes les pinces 29 de la position rétractée vers la position intermédiaire. Les paumelles 8 de la porte 4 sont alors toujours emprisonnées dans les pinces 29, mais la porte 4 n'est plus en contact étroit avec le bord supérieur de la cuve 2. Un pivotement de la porte autour de l'axe de pivotement formé par les pinces 29 est alors possible. Le moteur électrique du mécanisme 24 excentrique agencé du côté 6 de déchargement poursuit l'entraînement en rotation de ses coulisseaux 32 excentrées associés. Ces derniers entraînent alors le déplacement des pinces 29 agencées du côté 6 de déchargement en position totalement déployée, correspondant à une position libérée. La porte 4 est alors adaptée pour être ouverte du côté 6 de déchargement. L'unité 16 de commande peut alors autoriser l'actionnement des vérins formant les bras 7 télescopiques. Le déploiement des bras 7 assure l'ouverture de la porte 4 du côté 6 de déchargement.

Le passage de la porte de la position verrouillé à la position ouverte de chargement se déroule de manière analogue, à la différence qu'une fois en position intermédiaire, c'est le mécanisme excentrique agencé du côté de chargement qui poursuit l'entraînement en rotation de ses coulisseaux excentrés.

Le passage de la porte d'une position ouverte, par exemple de chargement, à la position fermée verrouillée implique séquentiellement, un actionnement de la pédale de chargement, une autorisation de fermeture délivrée par l'unité de commande, une vidange des vérins pneumatiques de manière à permettre la fermeture de la porte par gravité, une détection de la position intermédiaire de la porte, une mise en route des moteurs électriques des mécanismes excentriques pour positionner les pinces dans la position verrouillée.

Selon ce synopsis, la vidange des vérins occasionne le passage de la porte 4 de la position ouverte à une position dans laquelle elle recouvre la cuve 2. Les pinces 29 du côté de 5 chargement étant ouvertes, elles reçoivent les paumelles 8 de la porte 4 agencées en regard. La mise en route du moteur du mécanisme 23 excentrique du côté 5 de chargement permet par l'intermédiaire des coulisseaux 32 excentrés associés de refermer les pinces 29 du côté 5 de chargement de telle sorte que toutes les pinces 29 soient en position intermédiaire fermée. Les mécanismes 23 excentriques des deux côtés de la machine sont alors mis en route pour faire passer chaque pince 29 de sa position intermédiaire à la position, correspondant à la position verrouillée de la porte 4.

L'invention ne se limite pas aux seuls modes de réalisation décrits. En particulier, une machine à laver selon l'invention peut présenter une structure différente et être équipée de moyens de contrôle et de commande complémentaires aux fins de répondre à des nécessités spécifiques à certains instruments médicaux et/ou chirurgicaux.

Une machine selon l'invention peut en outre être programmée de manière à se comporter comme une machine à une porte et à un seul côté d'ouverture possible par porte.

Une machine selon l'invention peut en particulier comprendre plusieurs cuves, chacune étant équipée d'une porte bilatérale selon l'invention. Les cuves peuvent avoir un fonctionnement synchrone ou un fonctionnement asynchrone. Les cuves peuvent être agencées en ligne de façon à définir un côté de déchargement de la machine et un côté de chargement de la machine. Elles peuvent être agencées selon différentes configurations sans sortir du cadre de l'invention.

## Revendications

1. Machine à laver des instruments (1) médicaux et/ou chirurgicaux comprenant :
- au moins une cuve (2) de lavage adaptée pour recevoir des instruments (1) médicaux et/ou chirurgicaux, ladite (2) cuve étant délimitée par des parois (3) et comprenant une ouverture d'accès à la cuve pour la charger d'instruments (1) à laver ou la décharger d'instruments (1) lavés, ladite ouverture s'étendant dans un plan, dit plan (34) d'accès,
- une porte (4) agencée en regard de cette ouverture de ladite cuve (2) de lavage, ladite porte (4) étant montée sur la machine de façon à pouvoir passer d'une position, dite position de lavage, dans laquelle elle recouvre intégralement et de façon étanche ladite ouverture de ladite cuve (2) de lavage de manière à permettre un cycle de lavage à au moins une position, dite position ouverte, dans laquelle elle permet un accès à la cuve (2) de lavage pour charger des instruments (1) à laver ou décharger des instruments (1) lavés,
**caractérisée en ce que** ladite porte (4) est montée sur une articulation, dite articulation bilatérale, adaptée pour permettre une première ouverture latérale de la porte, dite ouverture de chargement, sur un premier côté, dit côté (5) de chargement de la cuve (2) de la machine, dans laquelle ladite porte (4) permet un accès à ladite cuve par ce côté (5) de chargement et interdit l'accès à ladite cuve (2) par le côté opposé, et pour permettre une seconde ouverture latérale, dite ouverture de déchargement, sur le côté opposé, dit côté (6) de déchargement de la cuve (2) de la machine à laver, dans laquelle ladite porte (4) permet un accès à ladite cuve par ce côté (6) de déchargement et interdit l'accès à la cuve (2) par ledit côté (5) de chargement.

2. Machine selon la revendication 1, **caractérisée en ce qu'**elle comprend ;
- des moyens de verrouillage à au moins deux états de ladite porte (4), un état verrouillé dans lequel lesdits moyens de verrouillage sont adaptés pour verrouiller ladite porte (4) en position de lavage de telle sorte qu'il soit impossible d'ouvrir ladite porte (4) au cours d'un cycle de lavage, et un état déverrouillé dans lequel lesdits moyens de verrouillage sont adaptés pour autoriser une ouverture de ladite porte (4),
- une unité (16) de commande desdits moyens de verrouillage adaptée pour pouvoir commander le verrouillage et le déverrouillage des moyens de verrouillage.

3. Machine selon la revendication 2, **caractérisée en ce qu'**elle comprend, associés à ladite unité (16) de commande :
- au moins un capteur de position de ladite porte, dit capteur (17) de porte, adapté pour détecter une position ouverte de ladite porte (4) et une position fermée de ladite porte (4),
- au moins un capteur de présence d'instruments médicaux et/ou chirurgicaux dans ladite cuve (2), dit capteur (18) de cuve,
- au moins un témoin (19) de lavage à deux états, un état lavé correspondant à une fin de cycle de lavage, et un état à laver, correspondant à un cycle de lavage à effectuer ou en cours.

4. Machine selon l'une des revendications 2 ou 3, **caractérisée en ce que** ladite unité (16) de commande est adaptée pour :
- commander le verrouillage desdits moyens de verrouillage si au moins ledit capteur (17) de porte détecte une porte (4) fermée, ledit capteur (18) de cuve détecte des instruments (1) dans ladite cuve (2) et ledit témoin (19) de lavage indique ledit état à laver,
- commander le déverrouillage desdits moyens de verrouillage dans les autres cas.

5. Machine selon l'une des revendications 2 à 4, **caractérisée en ce que** ladite unité (16) de commande est adaptée pour changer l'état dudit témoin (19) de lavage de l'état lavé à l'état à laver si au moins ledit capteur (18) de cuve ne détecte plus d'instruments (1) dans ladite cuve (2) de lavage.

6. Machine selon la revendication 5, **caractérisée en ce que** lesdits moyens de verrouillage comprennent trois états, un état verrouillé dans lequel ils sont adaptés pour verrouiller ladite porte (4) en position de lavage, un premier état déverrouillé, dit état déverrouillé de chargement dans lequel ils sont adaptés pour autoriser une ouverture de chargement de ladite porte (4), et un deuxième état déverrouillé, dit état déverrouillé de déchargement, dans lequel ils sont adaptés pour autoriser une ouverture de déchargement de ladite porte (4).

7. Machine selon les revendications 3 et 6, **caractérisée en ce que** ladite unité (16) de commande est adaptée pour :
- commander le passage des moyens de verrouillage dans l'état déverrouillé de chargement si au moins ledit capteur (18) de cuve détecte une absence d'instruments (1) de lavage et un capteur (17) de porte détecte une porte (4) fermée,
- commander le passage des moyens de verrouillage dans l'état déverrouillé de déchargement si au moins ledit témoin (19) de lavage indique ledit état lavé et un capteur (17) de porte détecte une porte (4) fermée.

8. Machine selon l'une des revendications 6 ou 7, **caractérisée en ce qu'**elle comprend une pédale, dite pédale de déchargement, agencée du côté de déchargement de la machine et adaptée pour solliciter une ouverture de déchargement de la porte (4) et une pédale, dite pédale de chargement, agencée du côté de chargement de la machine et adaptée pour solliciter une ouverture de chargement de la porte (4).

9. Machine selon l'une des revendications 1 à 8, **caractérisée en ce qu'**elle comprend un bâti (15) agencé autour de ladite cuve (2) comprenant une ouverture en regard de ladite ouverture de ladite cuve (2), et **en ce que** ladite porte (4) comprend des paumelles (8) agencées respectivement sur le bord de la porte (4) situé du côté (5) de chargement de la machine, une fois la porte (4) en position fermée, dit bord de chargement, et sur le bord de la porte (4) situé du côté (6) de déchargement, une fois la porte (4) en position fermée, dit tord de déchargement de la porte.

10. Machine à laver selon la revendication 9, **caractérisée en ce que** lesdits moyens de verrouillage comprennent au moins deux pinces (29) portés par le bâti (15) et agencées lorsque la porte (4) est en position fermée, respectivement en regard d'une paumelle (8) de la porte (4) agencée sur le bord
de chargement de la porte (4) et en regard d'une paumelle (8) de la porte (4) agencée sur le bord de déchargement de la porte (4), chaque pince (29) étant adaptée pour :
- dans une position, dite position verrouillée, correspondant audit état verrouillée, bloquer la paumelle (8) de la porte (4) agencée en regard de manière à interdire toute ouverture de la porte (4),
- dans une position, dite position libérée, correspondant à un état déverrouillé, permettre une libération de ladite paumelle (8) de la porte (4) agencée du côté de cette pince de manière à permettre une ouverture latérale de la porte (4) du côté de cette pince.

11. Machine selon la revendication 10, **caractérisée en ce que** chaque pince (29) comprend deux leviers (13, 14) munis respectivement de deux crochets (25, 26) conjugués adaptés pour pouvoir former une pince de saisie d'une paumelle (8) de ladite porte (4) et pour pouvoir être écarté l'un de l'autre de manière à autoriser la libération de cette paumelle (8) de ladite porte (4) et permettre une ouverture latérale de la porte (4).

12. Machine selon la revendication 11, **caractérisée en ce que** chaque pince (29) est montée sur le bâti (15) par l'intermédiaire d'au moins deux tourillons fixes par rapport au bâti, dit tourillons (23) de guidage et d'au moins un coulisseau mobile par rapport au bâti, dit coulisseau (32) de déplacement, ledit coulisseau (32) de déplacement étant adapté pour entraîner le déplacement desdits leviers (13, 14) de cette pince (29) dans un plan orthogonal audit plan d'accès (34) entre une position rétractée dans laquelle les leviers (13, 14) maintiennent la porte (4) en position de lavage, et au moins une position déployée dans laquelle les leviers (13, 14) autorisent une ouverture de la porte du côté de cette pince (29), la porte (4) est alors écartée de la cuve (2).

13. Machine selon la revendication 12, **caractérisée en ce que** chaque levier (13, 14) s'étend dans un plan orthogonal audit plan (34) d'accès et comprend une lumière oblongue, dite lumière (21) de déplacement, s'étendant selon une direction principale parallèle audit plan (34) d'accès et à travers laquelle est agencé, orthogonalement à ladite direction principale, ledit coulisseau (32) de déplacement, et deux lumières oblongues, dites lumières (22) de guidage, s'étendant selon une direction orthogonale audit plan (34) d'accès, agencées de part et d'autre de ladite lumière de déplacement et à travers lesquelles sont agencés lesdits tourillons (23) de guidage fixes par rapport audit bâti (15).

14. Machine selon la revendication 13, **caractérisée en ce que** ladite articulation de ladite porte (4) comprend sur chaque côté de (5) chargement et (6) de déchargement, un mécanisme, dit mécanisme (24) excentrique, comprenant un arbre (31) parallèle audit plan (34) d'accès adapté pour être entraîné en rotation par des moyens d'entraînement et à chaque extrémité duquel sont agencés deux coulisseaux (32) de déplacement excentrés, s'étendant selon une direction parallèle à la direction de l'arbre (31), chaque coulisseau (32) traversant lesdites lumières oblongues (21) de déplacement des leviers (13, 14) formant une pince (29) de sorte que la rotation excentrée desdits coulisseaux (32) de déplacement force un déplacement desdites pinces (29) orthogonalement audit plan (34) d'accès.

15. Machine selon l'une des revendications 13 ou 14, **caractérisée en ce que** pour chaque pince (29), l'une des lumières (22) oblongues de guidage d'un des leviers, dit levier (14) articulé, présente une portion inclinée par rapport à la lumière (22) de guidage du levier (13) en regard de telle sorte que ces lumières (22) de guidage des leviers (13, 14) formant cette pince (29) soient en partie non traversantes.

16. Machine selon l'une des revendications 12 à 15, **caractérisée en ce que** chaque coulisseau (32) de déplacement est adapté pour positionner chaque pince (29), dans une position intermédiaire déployée, dite position de pivot, entre ladite position rétractée et une position totalement déployée, et dans laquelle elle forme un gond de pivotement d'une paumelle (8) de la porte (4) enserrée dans cette pince (29).

17. Machine selon la revendication 14, **caractérisée en ce que** lesdits moyens d'entraînement en rotation desdits arbres (31) desdits mécanismes (24) excentriques sont associés à des moteurs électriques.

18. Machine selon l'une des revendications 10 à 17, **caractérisée en ce qu'**elle comprend des capteurs de position de chaque pince (29).

19. Machine selon l'une des revendications 1 à 18, **caractérisée en ce qu'**elle comprend deux bras (7) articulés télescopiques commandés agencés entre les bords latéraux de la porte (4) et la machine, lesdits bras (7) articulés étant adaptés pour pouvoir être déployés pour assurer une ouverture automatique de la porte (4) et pour pouvoir être rétractés pour permettre une fermeture de la porte (4).

20. Machine selon l'une des revendications 1 à 19, **caractérisée en ce que** ladite porte (4) est adaptée pour s'étendre, en position fermée, dans un plan horizontal, et **en ce que** ladite articulation bilatérale est agencée de sorte que lesdites ouvertures de chargement et de déchargement de ladite porte (4) soient des ouvertures vers le haut.

## Claims

1. A machine for washing medical and/or surgical instruments (1) comprising :
- at least one washing tank (2) designed to receive medical and/or surgical instruments (1), said tank (2) being defined by walls (3) and comprising an opening for access to the tank for loading instruments (1) to be washed into the tank or for unloading washed instruments (1) from the tank, said opening extending into a plane, called the access plane (34),
- a door (4) arranged opposite said opening of said washing tank (2), said door (4) being mounted on the machine so as to be able to pass from a position, called the washing position, in which it entirely and sealingly covers said opening of said washing tank (2) so as to permit a washing cycle, into at least one position, called the open position, in which it permits access to the washing tank (2) to load the instruments (1) to be washed or to unload the washed instruments (1),
**characterized in that** said door (4) is mounted on a hinge, called a bilateral hinge, designed to allow a first lateral opening of the door, called the opening for loading, on a first side, called the loading side (5) of the tank (2) of the machine, in which said door (4) permits access to said tank from said loading side (5) and prevents access to said tank (2) from the opposing side, and to permit a second lateral opening, called the opening for unloading, on the opposing side, called the unloading side (6) of the tank (2) of the washing machine, in which said door (4) permits access to said tank from said unloading side (6) and prevents access to the tank (2) from said loading side (5).

2. Machine according to claim 1, **characterized in that** it comprises:
- means for locking in at least two states of said door (4), a locked state in which said locking means are designed to lock said door (4) in the washing position such that it is impossible to open said door (4) during a washing cycle, and an unlocked state in which said locking means are designed to allow an opening of said door (4),
- a control unit (16) for said locking means designed to be able to control the locking and unlocking of the locking means.

3. Machine according to claim 2, **characterized in that** it comprises, associated with said control unit (16) :
- at least one positioning sensor for said door, called a door sensor (17), designed to detect an open position of said door (4) and a closed position of said door (4),
- at least one sensor for detecting the presence of medical and/or surgical instruments in said tank (2) called the tank sensor (18),
- at least one washing indicator (19) in two states, a washed state corresponding to the end of the washing cycle and a state to be washed corresponding to a washing cycle to be carried out or underway.

4. Machine according to one of claims 2 or 3, **characterized in that** said control unit (16) is designed to:
- control the locking of said locking means if at least said door sensor (17) detects a closed door (4), said tank sensor (18) detects instruments (1) in said tank (2) and said washing indicator (19) indicates said state to be washed,
- control the unlocking of said locking means in the other cases.

5. Machine according to one of claims 2 to 4, **characterized in that** said control unit (16) is designed to change the state of said washing indicator (19) from the washed state to the state to be washed if at least said tank sensor (18) no longer detects instruments (1) in said washing tank (2).

6. Machine according to claim 5, **characterized in that** said locking means comprise three states, a locked state in which they are designed to lock said door (4) in the washing position, a first unlocked state, called the unlocked loading state in which they are designed to permit an opening of said door (4) for loading, and a second unlocked state, called the unlocked unloading state, in which they are designed to permit an opening of said door (4) for unloading.

7. Machine according to claims 3 and 6, **characterized in that** said control unit (16) is designed to:
- control the passage of the locking means into the unlocked loading state if at least said tank sensor (18) detects an absence of instruments (1) for washing and a door sensor (17) detects a closed door (4),
- control the passage of the locking means into the unlocked unloading state if at least said washing indicator (19) indicates said washed state and a door sensor (17) detects a closed door (4).

8. Machine according to one of claims 6 or 7, **characterized in that** it comprises a pedal, called the unloading pedal, arranged on the unloading side of the machine and designed to activate an opening of the door (4) for unloading and a pedal, called the loading pedal, arranged on the loading side of the machine and designed to activate an opening of the door (4) for loading.

9. Machine according to one of claims 1 to 8, **characterized in that** it comprises a frame (15) arranged about said tank (2) and comprising an opening opposite said opening of said tank (2) and **in that** said door (4) comprises hinge plates (8) arranged respectively on the edge of the door (4) located on the loading side (5) of the machine, once the door (4) is in the closed position, called the loading edge, and on the edge of the door (4) located on the unloading side (6), once the door (4) is in the closed position, called the unloading edge of the door.

10. Washing machine according to claim 9, **characterized in that** said locking means comprise at least two claws (29) carried by the frame (15) and arranged, when the door (4) is in the closed position, respectively opposite a hinge plate (8) of the door (4) arranged on the loading edge of the door (4) and opposite a hinge plate (8) of the door (4) arranged on the unloading edge of the door (4), each claw (29) being designed:
- in one position, called the locked position, corresponding to said locked state, to block the hinge plate (8) of the door (4) arranged opposite so as to prevent any opening of the door (4),
- in one position, called the released position, corresponding to an unlocked state, to permit a release of said hinge plate (8) of the door (4) arranged on said claw side so as to permit a lateral opening of the door (4) on said claw side.

11. Machine according to claim 10, **characterized in that** each claw (29) comprises two levers (13, 14) respectively provided with two hooks (25, 26) which are joined together and designed to be able to form a claw for clamping a hinge plate (8) of said door (4) and to be able to be separated from one another so as to allow the release of said hinge plate (8) of said door (4) and to permit a lateral opening of the door (4).

12. Machine according to claim 11, **characterized in that** each claw (29) is mounted on the frame (15) by means of at least two journals which are fixed relative to the frame, called guide journals (23) and at least one slider which is mobile relative to the frame, called the displacement slider (32), said displacement slider (32) being designed to cause the displacement of said levers (13, 14) of said claw (29) in a plane at right angles to said access plane (34) between a retracted position in which the levers (13, 14) maintain the door (4) in a washing position, and at least one deployed position in which the levers (13, 14) allow an opening of the door on said claw (29) side, the door (4) being thus moved away from the tank (2).

13. Machine according to claim 12, **characterized in that** each lever (13, 14) extends in a plane at right angles to said access plane (34) and comprises an oblong aperture, called the displacement aperture (21), extending in a principal direction parallel to said access plane (34) and through which is arranged, at right angles to said principal direction, said displacement slider (32) and two oblong apertures, called guide apertures (22), extending in a direction at right angles to said access plane (34), arranged on both sides of said displacement aperture and through which are arranged said guide journals (23) which are fixed relative to said frame (15).

14. Machine according to claim 13, **characterized in that** said hinge of said door (4) comprises on each loading and unloading side (5, 6), a mechanism, called an eccentric mechanism (24), comprising a shaft (31) which is parallel to said access plane (34) and designed to be driven in rotation by driving means and at each end of which are arranged two eccentric displacement sliders (32), extending in a direction parallel to the direction of the shaft (31), each slider (32) passing through said oblong displacement apertures (21) of the levers (13, 14) forming a claw (29) such that the eccentric rotation of said displacement sliders (32) forces a displacement of said claws (29) at right angles to said access plane (34).

15. Machine according to one of claims 13 or 14, **characterized in that** for each claw (29), one of the oblong guide apertures (22) for one of the levers, called the articulated lever (14), has a portion which is inclined relative to the guide aperture (22) of the opposite lever (13) such that said guide apertures (22) of the levers (13, 14) forming this claw (29) are partially blind.

16. Machine according to one of claims 12 to 15, **characterized in that** each displacement slider (32) is designed to position each claw (29), in an intermediate deployed position, called the pivoted position, between said retracted position and a fully deployed position, and in which it forms a pivot pin of a hinge plate (8) of the door (4) clamped in said claw (29).

17. Machine according to claim 14, **characterized in that** said means for driving said shafts (31) of said eccentric mechanisms (24) in rotation are associated with electric motors.

18. Machine according to one of claims 10 to 17, **characterized in that** it comprises positioning sensors for each claw (29).

19. Machine according to one of claims 1 to 18, **characterized in that** it comprises two controlled telescopic articulated arms (7) arranged between the lateral edges of the door (4) and the machine, said articulated arms (7) being designed to be deployed to ensure automatic opening of the door (4) and to be able to be retracted to allow a closing of the door (4).

20. Machine according to one of claims 1 to 19, **characterized in that** said door (4) is designed to extend in the closed position, in a horizontal plane, and **in that** said bilateral hinge is arranged such that said loading and unloading apertures of said door (4) are openings at the top.

## Patentansprüche

1. Waschmaschine für medizinische und / oder chirurgische Instrumente, umfassend:
- wenigstens ein Waschbecken (2), das zur Aufnahme der medizinischen und / oder chirurgischen Instrumente (1) geeignet ist, wobei das genannte Becken (2) durch Wände (3) begrenzt ist und eine Zugangsöffnung zum Becken umfasst, um es mit zu waschenden Instrumenten (1) zu laden oder gewaschene Instrumente (1) daraus zu entladen, wobei die genannte Öffnung sich in einer Ebene, bezeichnet als Zugangsebene (34), erstreckt,
- eine Tür (4), die gegenüber dieser Öffnung des genannten Waschbeckens (2) angeordnet ist, wobei die genannte Tür (4) an der Maschine derart angebracht ist, dass sie von einer Position, bezeichnet als Waschposition, in der sie die genannte Öffnung des genannten Waschbeckens (2) komplett und abgedichtet derart abdeckt, dass ein Waschzyklus mit wenigstens einer Position, bezeichnet als offene Position, in der sie einen Zugang zum Waschbecken (2) zum Laden der zu waschenden Instrumente (1) oder Entladen der gewaschenen Instrumente (1) erlaubt, ermöglicht wird,
**dadurch gekennzeichnet, dass** die genannte Tür (4) auf eine Artikulation, bezeichnet als bilaterale Artikulation, die geeignet ist, um eine erste laterale Öffnung der Tür, bezeichnet als Beladeöffnung, auf einer ersten Seite, bezeichnet als Beladeseite (5) des Beckens (2) der Maschine zu erlauben, in der die genannte Tür (4) einen Zugang zu dem genannten Beladebecken (5) erlaubt und den Zugang zu dem genannten Becken (2) von der entgegengesetzten Seite verbietet, und um eine zweite laterale Öffnung, bezeichnet als Entladeöffnung, auf der entgegengesetzten Seite, bezeichnet als Entladeseite (6) des Beckens (2) der Waschmaschine, zu erlauben, in der die genannte Tür (4) einen Zugang zu dem genannten Becken durch diese Entladeseite (6) erlaubt und den Zugang zum Becken (2) durch die genannte Beladeseite (5) verbietet, angebracht ist.

2. Maschine gemäß Anspruch 1, **dadurch gekennzeichnet, dass** sie umfasst:
- Verriegelungsmittel mit wenigstens zwei Zuständen der genannten Tür (4), einem verriegelten Zustand, in dem die genannten Verriegelungsmittel geeignet sind, um die genannte Tür (4) in der Waschposition derart zu verriegeln, dass es unmöglich ist, die genannte Tür (4) im Verlauf eines Waschzyklus zu öffnen, und ein entriegelter Zustand, in dem die genannten Verriegelungsmittel geeignet sind, um eine Öffnung der genannten Tür (4) zuzulassen,
- eine Steuereinheit (16) der genannten Verriegelungsmittel, die geeignet ist, um die Verriegelung und Entriegelung der Verriegelungsmittel steuern zu können.

3. Maschine gemäß Anspruch 2, **dadurch gekennzeichnet, dass** sie in Verbindung mit der genannten Steuereinheit (16) umfasst:
- wenigstens einen Positionssensor der genannten Tür, bezeichnet als Türsensor (17), der geeignet ist, um eine offene Position der genannten Tür (4) und eine geschlossene Position der genannten Tür (4) festzustellen.
- wenigstens einen Präsenzsensor für medizinische und / oder chirurgische Instrumente in dem genannten Becken (2), bezeichnet als Beckensensor (18),
- wenigstens eine Waschanzeige (19) mit zwei Zuständen, wobei ein gewaschener Zustand einem Ende des Waschzyklus entspricht und ein zu waschender Zustand einem noch durchzuführenden oder einem Waschzyklus in Bearbeitung entspricht.

4. Maschine gemäß Anspruch 2 oder 3, **dadurch gekennzeichnet, dass** die genannte Steuereinheit (16) geeignet ist, um:
- die Verriegelung der genannten Verriegelungsmittel zu steuern, wenn wenigstens der genannte Türsender (17) eine geschlossene Tür (4) feststellt, wobei der genannte Beckensensor (18) Instrumente (1) in dem genannten Becken (2) feststellt und die genannte Waschanzeige (19) den genannten zu waschenden Zustand anzeigt;
- die Entriegelung der genannten Verriegelungsmittel in den anderen Fällen zu steuern.

5. Maschine gemäß Anspruch 2 bis 4, **dadurch gekennzeichnet, dass** die genannte Steuereinheit (16) geeignet ist, um den Zustand der genannten Waschanzeige (19) vom gewaschenen Zustand zum zu waschenden Zustand zu ändern, wenn wenigstens der genannte Beckensensor (18) keine Instrumente (1) mehr in dem genannten Waschbecken ((2) feststellt.

6. Maschine gemäß Anspruch 5, **dadurch gekennzeichnet, dass** die genannten Verriegelungsmittel drei Zustände umfassen, einen verriegelten Zustand, in dem sie geeignet sind, um die genannte Tür (4) in der Waschposition zu verriegeln, einen ersten entriegelten Zustand, bezeichnet als entriegelter Entladezustand, indem sie geeignet sind, um eine Ladeöffnung der genannten Tür (4) zuzulassen, und einen zweiten entriegelten Zustand, bezeichnet als entriegelter Entladezustand, in dem sie geeignet sind, um eine Entladeöffnung der genannten Tür (4) zuzulassen.

7. Maschine gemäß Anspruch 3 und 6, **dadurch gekennzeichnet, dass** die genannte Steuereinheit (16) geeignet ist, um:
- den Übergang der Verriegelungsmittel in den entriegelten Entladezustand zu steuern, wenn wenigstens der genannte Beckensensor (18) ein Fehlen von Waschinstrumenten (1) und ein Türsensor (17) eine geschlossene Tür (4) feststellt,
- den Übergang der Verriegelungsmittel in den entriegelten Entladezustand zu steuern, wenn wenigstens die genannte Waschanzeige (19) den genannten gewaschenen Zustand anzeigt und ein Türsensor (17) eine geschlossene Tür (4) feststellt.

8. Maschine gemäß Anspruch 6 oder 7, **dadurch gekennzeichnet, dass** sie eine Pedale, bezeichnet als Entladepedale, umfasst, die an der Entladeseite der Maschine angeordnet ist und geeignet ist, um eine Entladeöffnung der Tür (4) anzusprechen, und eine Pedale, bezeichnet als Ladepedale, die auf der Beladeseite der Maschine angeordnet ist und geeignet ist, um eine Ladeöffnung der Tür (4) anzusprechen.

9. Maschine gemäß Anspruch 1 bis 8, **dadurch gekennzeichnet, dass** sie ein Gestell (15) umfasst, das um das genannte Becken (2) angeordnet ist, das eine Öffnung gegenüber der genannten Öffnung des genannten Beckens (2) umfasst, und dass die genannte Tür (4) Türbänder (8) umfasst, die jeweils auf dem Rand der Tür (4) angeordnet sind, der sich auf der Beladeseite (5) der Maschine befindet, sobald die Tür (4) in der Position, bezeichnet als Laderand, geschlossen ist, und auf dem Rand der Tür (4), der sich auf der Entladeseite (6) befindet, sobald sich die Tür (4) in geschlossener Position befindet, bezeichnet als Entladerand der Tür.

10. Waschmaschine gemäß Anspruch 9, **dadurch gekennzeichnet, dass** die genannten Verriegelungsmittel wenigstens zwei Klemmen (29) umfassen, die vom Gestell (15) getragen werden und wenn die Tür (4) sich in geschlossener Position befindet, jeweils gegenüber eines am Laderand der Tür (4) angeordneten Türbandes (8) der Tür (4) und gegenüber einem am Entladerand der Tür (49 angeordneten Türband (8) der Tür (4) angeordnet sind, wobei jede Klemme (29) geeignet ist, um:
- in einer Position, bezeichnet als verriegelte Position, die dem verriegelten Zustand entspricht, das Türband (8) der Tür (4) zu verriegeln, das derart gegenüber angeordnet ist, um, jegliche Öffnung der Tür (4) zu verbieten,
- in einer Position, bezeichnet als freigegebene Position, die einem entriegelten Zustand entspricht, eine Freigabe des genannten Türbandes (8) der Tür (4) zu erlauben, das auf der Seite dieser Klemme derart angeordnet ist, dass eine laterale Öffnung der Tür (4) auf der Seite dieser Klemme erlaubt wird,

11. Maschine gemäß Anspruch 10, **dadurch gekennzeichnet, dass** jede Klemme (29) zwei Hebel (13, 14) umfasst, die jeweils mit zwei Haken (25, 26) versehen sind, die gekoppelt geeignet sind, um eine Greifzange eines Türbandes (8) der genannten Tür (4) bilden zu können und um voneinander derart beabstandet sein zu können, dass die Freisetzung dieses Türbandes (8) von der genannten Tür (4) zugelassen wird und eine laterale Öffnung der Tür (4) zu erlauben.

12. Maschine gemäß Anspruch 11, **dadurch gekennzeichnet, dass** jede Klemme (29) auf dem Gestell (15) durch wenigstens zwei im Verhältnis zum Gestell feste Zapfen, bezeichnet als Führungszapfen (23), und wenigstens einen im Verhältnis zum Gestell mobilen Schieber, bezeichnet als Verschiebungsschieber (32), montiert ist, wobei der genannte Verschiebungsschieber (32) geeignet ist, um die Verschiebung der genannten Hebel (13, 14) dieser Klemme (29) in einer zur Zugangsebene (34) orthogonalen Ebene zwischen einer eingezogenen Position, in der die Hebel (13, 14) die Tür (4) in der Waschposition halten, und wenigstens einer ausgefahrenen Position, in der die Hebel (13, 14) eine Öffnung der Tür auf der Seite dieser Klemme (29) zulassen, anzutreiben, die Tür (4) ist dann vom Becken (2) beabstandet.

13. Maschine gemäß Anspruch 12, **dadurch gekennzeichnet, dass** jeder Hebel (13, 14) sich in einer orthogonalen Ebene zur Zugangsebene (34) erstreckt und eine längliche Öffnung, bezeichnet als Verschiebungsöffnung (21) umfasst, die sich gemäß einer zur genannten Zugangsebene (34) parallelen Hauptrichtung erstreckt und durch die orthogonal zur genannten Hauptrichtung der genannte Verschiebungsschieber (32) angeordnet ist, und zwei längliche Öffnungen, bezeichnet als Führungsöffnungen (22), die sich gemäß einer zur Zugangsebene (34) orthogonalen Richtung erstrecken und auf jeder Seite der genannten Verschiebungsöffnung angeordnet sind und über die die genannten, im Verhältnis zum genannten Gestell (15) festen Führungszapfen (23) angeordnet sind.

14. Maschine gemäß Anspruch 13, **dadurch gekennzeichnet, dass** die genannte Artikulation der genannten Tür (4) auf jeder Ladeseite (5) und Entladeseite (6) einen Mechanismus, bezeichnet als exzentrischer Mechanismus (24) umfasst, umfassend eine zur Zugangsebene (34) parallele Welle, die geeignet ist, um durch Antriebsmittel in Rotation angetrieben zu werden und an deren Enden jeweils zwei exzentrische Verschiebunsschieber (32) angeordnet sind, die sich gemäß einer zur Richtung der Welle (31) parallelen Richtung erstrecken, wobei jeder Schieber (32) die genannten länglichen Verschiebungsöffnungen (21) der Hebel (13, 14) durchquert, die eine Klemme (29) derart bilden, dass die exzentrische Rotation der genannten Verschiebungsschieber (32) eine Verschiebung der genannten Klemmen (29) orthogonal zur genannten Zugangsebene (34) erzwingt.

15. Maschine gemäß Anspruch 13 oder 14, **dadurch gekennzeichnet, dass** für jede Klemme (29) eine der länglichen Führungsöffnungen (22) eines der Hebel, bezeichnet als artikulierter Hebel (14), einen im Verhältnis zur Führungsöffnung (22) des Hebels (13) geneigten Abschnitts gegenüber derart aufweist, dass diese Führungsöffnungen (22) der diese Klemme (29) bildenden Hebel (13, 14) zum Teil nicht durchquerend sind.

16. Maschine gemäß Anspruch 12 bis 15, **dadurch gekennzeichnet, dass** jeder Verschiebungsschieber (32) geeignet ist, um jede Klemme (29) in einer ausgefahrenen Zwischenposition, bezeichnet als Schwenkposition, zwischen der genannten eingefahrenen Position und einer komplett ausgefahrenen Position zu positionieren, in der sie eine Schwenkangel eines Türbandes (8) der in dieser Klemme (29) eingeklemmten Tür (4) bildet.

17. Maschine gemäß Anspruch 14, **dadurch gekennzeichnet, dass** die genannten Antriebsmittel in Rotation der genannten Wellen (31) der genannten exzentrischen Mechanismen (24) ebenfalls Elektromotoren zugeordnet sind.

18. Maschine gemäß Anspruch 10 bis 17, **dadurch gekennzeichnet, dass** sie Positionssensoren jeder Klemme (29) umfasst.

19. Maschine gemäß Anspruch 1 bis 18, **dadurch gekennzeichnet, dass** sie zwei artikulierte teleskopisch gesteuerte Arme (7) umfasst, die zwischen den lateralen Rändern der Tür (4) und der Maschine angeordnet sind, wobei die genannten artikulierten Arme (7) geeignet sind, um ausgefahren werden zu können, um eine automatische Öffnung der Tür (4) zu gewährleisten und um eingefahren werden zu können, um ein Schließen der Tür (4) zu erlauben.

20. Maschine gemäß Anspruch 1 bis 19, **dadurch gekennzeichnet, dass** die genannte Tür (4) geeignet ist, um sich in geschlossener Position in einer horizontalen Ebene zu erstrecken, und dass die genannte bilaterale Artikulation derart angeordnet ist, dass die genannten Lade- und Entladeöffnungen der genannten Tür (4) Öffnungen nach oben sind.
